# FASCICULE DE BREVET EUROPEEN

(11) **EP 1 412 484 B1**
(45) Date de publication et mention de la délivrance du brevet: **20.10.2010**
(21) Numéro de dépôt: 02772482.2
(22) Date de dépôt: 02.08.2002
(51) Int. Cl.: C12N 5/071, C12N 5/077, A61K 35/32, A61K 35/36, C12Q 1/02

(54) **UTILISATION D'INHIBITEURS DES LYSYL OXYDASES POUR LA CULTURE CELLULAIRE ET LE GENIE TISSULAIRE**
VERWENDUNG VON LYSYLOXYDASE INHIBITOREN ZUR ZELLKULTUR UND GEWEBEAUFBAU
USE OF LYSYL OXIDASE INHIBITORS FOR CELL CULTURE AND TISSUE ENGINEERING

(30) Priorité: 03.08.2001 FR 0110443
(43) Date de publication de la demande: 28.04.2004
(73) Titulaire: CENTRE NATIONAL DE LA RECHERCHE SCIENTIFIQUE (CNRS), 75794 Paris Cedex 16 (FR); UNIVERSITE CLAUDE BERNARD - LYON 1, 69622 Villeurbanne Cédex (FR)
(72) Inventeur: FARJANEL, Jean, F-69003 Lyon (FR); DAMOUR née BAUDOUX, Odile, F-69230 St Genis Laval (FR); SOMMER, Pascal, F-69230 St Genis-Laval (FR); BRAYE, Fabienne, 69780 Saint Pierre de Chandieu (FR); FESSY, Michel-Henri, F-69390 Millery (FR)
(74) Mandataire: Grosset-Fournier, Chantal Catherine
(86) Numéro de dépôt international: PCT/FR2002/002789
(87) Numéro de publication internationale: WO 2003/014333

(56) Documents cités:
- EP-A- 0 731 163
- WO-A-00/29553
- WO-A-00/51527
- US-A- 5 521 087
- BEEKMAN B ET AL: "Synthesis of collagen by bovine chondrocytes cultured in alginate; posttranslational modifications and cell-matrix interaction." EXPERIMENTAL CELL RESEARCH, vol. 237, no. 1, 25 novembre 1997 (1997-11-25), pages 135-141, XP002201922 ISSN: 0014-4827
- MODIS L ET AL: "Proteoglycan biosynthesis is stimulated by D-penicillamine in chondrifying high density cell cultures" EXPERIMENTAL PATHOLOGY, vol. 35, no. 3, 1988, pages 159-176, XP008004196 ISSN: 0232-1513
- AHSAN T ET AL: "Integrative cartilage repair: Inhibition by beta-aminopropionitrile." JOURNAL OF ORTHOPAEDIC RESEARCH, vol. 17, no. 6, novembre 1999 (1999-11), pages 850-857, XP008004199 ISSN: 0736-0266
- WOODLEY D T ET AL: "Collagen telopeptides cross-linking sites play a role in collagen gel lattice contraction" JOURNAL OF INVESTIGATIVE DERMATOLOGY, vol. 97, no. 3, septembre 1991 (1991-09), pages 580-585, XP008004198 ISSN: 0022-202X
- KAGAN H M: "Intra- and extracellular enzymes of collagen biosynthesis as biological and chemical targets in the control of fibrosis." ACTA TROPICA, vol. 77, no. 1, 2000, pages 147-152, XP002201923 ISSN: 0001-706X cité dans la demande
- OMORI K ET AL: "Regulation of the expression of lysyl oxidase mRNA in cultured rabbit retinal pigment epithelium cells." MATRIX BIOLOGY, vol. 21, no. 4, juin 2002 (2002-06), pages 337-348, XP002223343 ISSN: 0945-053X

## Description

L'invention a pour objet l'utilisation d'inhibiteurs des lysyl oxydases dans le cadre de la mise en oeuvre de procédés de culture *in vitro* de cellules susceptibles d'être utilisées en thérapie tissulaire, ou cellulaire, ou en pharmacologie expérimentale.

Les lysyl oxydases sont des amine oxydases qui induisent la réticulation des collagènes et de l'élastine en catalysant la désamination oxydative de résidus lysyls et hydroxylysyls en α-aminoadipic-S-semialdéhydes correspondants (pour revue, Smith-Mango & Kagan, Matrix Biology 1998, 16 : 387): **RCH₂NH₂ + O₂ + H₂O** → **RCHO + NH₃ + H₂O₂**. Les résidus aldéhydes formés vont subir une série de condensations spontanées avec d'autres fonctions aldéhydes ou amines non modifiées voisines, conduisant à des liaisons intra et inter-moléculaires. Ces condensations sont à l'origine des pontages intra et intermoléculaires des collagènes et de l'élastine. Parmi les inhibiteurs directs de l'activité LO, qui agissent sur le site actif de l'enzyme, le βAPN en est le plus couramment utilisé (voir revue de H.M.Kagan Acta Tropica 77 (2000) 147-152).

La réticulation dépendante des LO des collagènes et d'élastine est un paramètre essentiel de la formation des tissus et implants néosynthétisés *in vitro* : L'absence de réticulation ne permet pas la constitution de tissus résistants, trop de réticulation (ou une réticulation chimique inappropriée) entraînent une trop grande rigidité et une rétractation des tissus.

La présente invention découle de la mise en évidence par les Inventeurs du fait que l'adjonction d'un inhibiteur spécifique des LO à des chondrocytes, cellules spécifiques du cartilage, permet d'annuler la dédifférenciation de ces cellules. En effet, le problème majeur de la formation d'implants de cartilage par les chondrocytes est que ceux-ci se dédifférencient, et perdent leur potentiel à fabriquer du collagène de cartilage (collagènes de type cartilage : II, IX, XI et X). Sans cet inhibiteur, les chondrocytes dédifférenciés synthétisent des types de collagènes anormaux dans le cartilage sain (I, III). L'adjonction temporaire de l'inhibiteur de LO a donc un effet important et réversible sur la formation du cartilage, dont les propriétés physiques spécifiques sont liées à la présence des collagènes qui le constituent.

Il est connu que la culture en gel d'alginate permet d'éviter la dédifférenciation des chondrocytes et que l'addition de β-aminopropionitrile (βAPN) augmente la synthèse globale du collagène et en réduit la réticulation. (B. Beekman et coll., Experimental Cell Research 1997, 237(1):135-141) Il n'est pas mis en évidence d'effet direct du βAPN sur la différenciation des cellules et l'absence d'étude détaillée des sous-types de collagène ne permet pas d'exclure la présence minoritaire de collagène I. D'autres études ont montré que l'application de βAPN sur un explant de cartilage déjà formé en réduisait la réticulation et concluent à l'intérêt de ne pas utiliser de βAPN sur un explant de cartilage pour la réparation tissulaire. (Ahsan T et coll., Journal of Orthopedic Research 1999, 17(6):850-857).

De plus, les Inventeurs ont également mis en évidence que l'adjonction du même inhibiteur des LO lors de la constitution d'un modèle de peau reconstruite se traduit aussi par un effet sur le phénotype des cellules. Les composants cellulaires du modèle testé de peau reconstruite sont des fibroblastes et des kératinocytes. Les fibroblastes synthétisent notamment les collagènes fibrillaires (type I et III) et l'élastine du derme, alors que les kératinocytes se différencient pour former toutes les couches de l'épiderme jusqu'à la couche cornée assurant la fonction de barrière. En présence de l'inhibiteur de LO, les propriétés de la peau reconstruite sont modifiées. On observe que l'absence de réticulation des collagènes permet une meilleure colonisation du support éponge par les fibroblastes et une augmentation de la formation de la matrice extracellulaire, ainsi qu'une diminution *in vitro* de la rétraction de la peau reconstruite. D'autre part, on observe une meilleure organisation de l'épiderme, avec une couche granuleuse très bien formée. L'adjonction temporaire d'inhibiteur des LO a donc un effet positif sur la gestion de la formation et l'organisation de la peau reconstruite en facilitant la préparation en grande quantité de ce matériel.

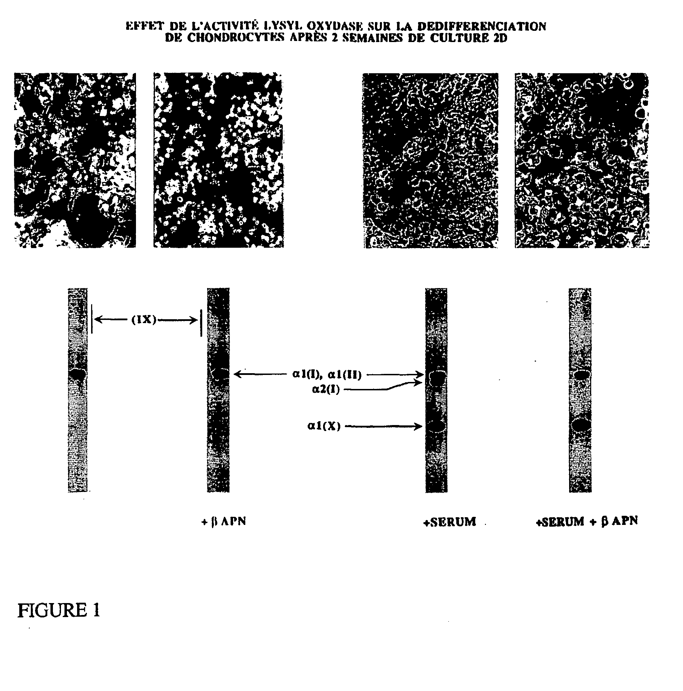

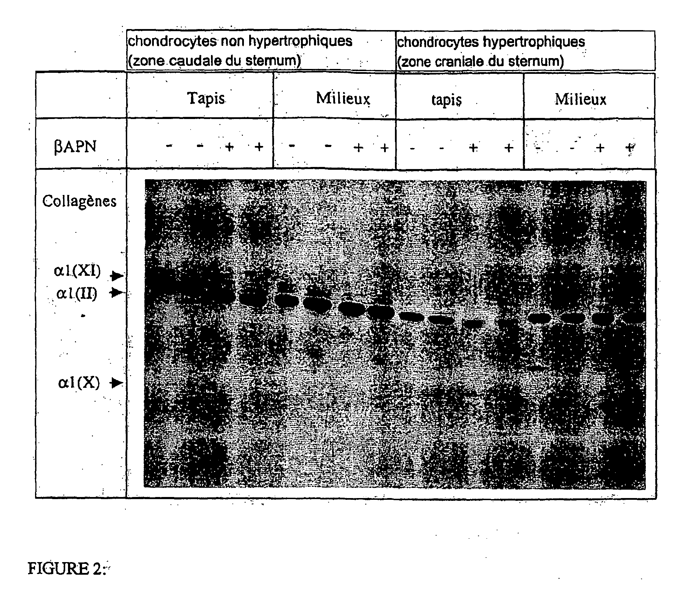

A partir de biopsies de cartilage, et en présence d'inhibiteurs de LO, les Inventeurs ont démontré qu'il devient possible de multiplier des chondrocytes sur un support plastique en 2 dimensions en présence de sérum du donneur selon les conditions classiques, tout en obtenant la production d'une matrice extracellulaire dénuée de tout collagène anormal, même après 2 semaines de culture, et malgré une solubilisation accrue des collagènes synthétisés dans le milieu de culture (Fig 1, 2). Une telle matrice voit sa fluidité diminuer au cours du temps de culture : on choisit le temps de culture optimal en fonction de la quantité de matrice produite et de sa fluidité recherchées. Il est alors inutile de recourir à l'emploi de support exogène dont, ultérieurement, le rejet ou une résorption défectueuse éventuelles annuleraient les propriétés favorables déjà obtenues.

En l'absence de l'inhibiteur type de l'activité LO, le βAPN, la matrice extracellulaire est très appauvrie en collagènes de type cartilage, et majoritairement constituée de collagènes de type I ainsi que, pour une faible part, de collagène de type III, c'est à dire de collagènes qui ne participent pas à l'élaboration du cartilage. De plus, la matrice présente une absence de fluidité incompatible avec le recours éventuel à des injections par seringue.

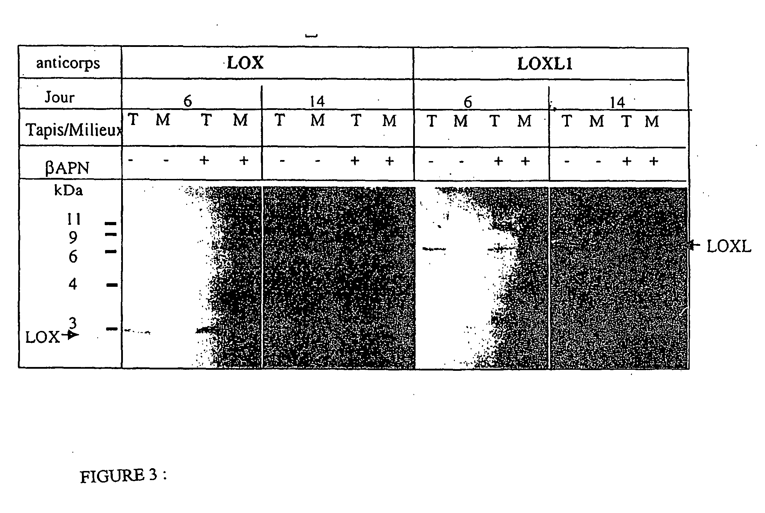

L'expression de plusieurs isoformes de lysyl oxydase (LOX et LOL1 au moins) par les chondrocytes est manifeste, mais n'est pas modifiée par le traitement par le βAPN (fig 3) qui n'inhibe que l'activité de l'enzyme (tableau I).

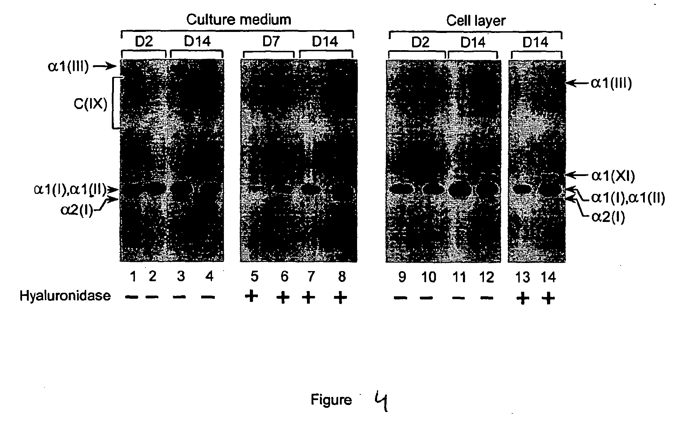

L'adjonction de βAPN, utilisée à différentes concentrations (entre 50 ou 200µg/ml), permet la formation de peaux reconstruites humaines dont la rétraction peut être diminuée in *vitro.* De plus, le βAPN à 50 µg/ml améliore la colonisation de l'éponge par les fibroblastes et l'extension de la synthèse de la matrice extracellulaire. Il améliore aussi l'organisation de l'épiderme, avec une couche granuleuse très bien formée qui génère une couche cornée très structurée. Cet effet sur la différenciation de l'épiderme et la diminution de la rétraction réduisent la formation de feuillets qui se détachent et peuvent faciliter la préparation de peau reconstruite (fig 4).

L'utilisation d'inhibiteurs de l'activité LO peut s'appliquer à toute culture de cellules aux fins d'obtenir tout implant tissulaire, puisque les collagènes et les LO sont des constituants et des intervenants ubiquitaires de tous les tissus animaux. Les conditions des cultures peuvent cependant varier en fonction des types cellulaires utilisés et des substrats ciblés.

L'invention a pour objet l'utilisation d'inhibiteurs directs ou indirects des lysyl oxydases (LO), en tant qu'inhibiteurs de la dédifférenciation des cellules en culture *in vitro,* en vue du maintien quasiment constant du phénotype desdites cellules dans le cadre de la mise en oeuvre de procédés de culture *in vitro* de ces dernières, et ce pendant la quasi totalité du temps de culture.

A ce titre, l'invention a plus particulièrement pour objet l'utilisation d'inhibiteurs susmentionnés pour la préparation de cellules différenciées dont le phénotype est préservé.

L'invention concerne plus particulièrement encore l'utilisation d'inhibiteurs susmentionnés pour la préparation d'une matrice cellulaire, constituée de cellules différenciées cultivées en présence desdits inhibiteurs, et du milieu extracellulaire conditionné par lesdites cellules et au contact de ladite matrice, ladite matrice cellulaire étant susceptible de pouvoir être utilisée en tant que tissus ou implants tissulaires.

L'invention a également pour objet l'utilisation susmentionnée, d'inhibiteurs directs des LO choisis parmi :
les amines primaires réagissant avec le groupe carbonyle du site actif des LO, et plus particulièrement celles donnant, après liaison au carbonyle, un produit stabilisé par résonance, telles que les amines primaires suivantes :
   - l'éthylènediamine,
   - les aminonitriles, tels que le β-aminopropionitrile (β-APN), ou le 2-nitroéthylamine,
   - les haloamines insaturées ou saturées, telles que la 2-bromo-éthylamine, 2-chloroéthylamine, 2-trifluoroéthylamine, 3-bromopropylamine, les p-halobenzylamines,
   - la sélénohomocystéine lactone,

L'invention a plus particulièrement pour objet l'utilisation du β-APN aux fins susmentionnées.

L'invention concerne également l'utilisation d'inhibiteurs de LO tels que définis ci-dessus, pour la mise en oeuvre de procédés de culture *in vitro* de toutes cellules d'origine humaine ou animale, lesdites cellules sont maintenues à un phénotype de différenciation constant, et sont choisies notamment parmi les chondrocytes des cartilages, les cellules de la cornée, les cellules de la peau (tels que les fibroblastes de derme, et kératinocytes de l'épiderme), les cellules endothéliales des vaisseaux, les ostéoblastes des os, les hépatocytes, les cellules rénales, les cellules musculaires, les cellules souches ou pluripotentes.

L'invention a également pour objet l'utilisation d'inhibiteurs de LO tels que définis ci-dessus, pour la mise en oeuvre de procédés de *culture in vitro* de cellules, en vue de l'obtention de cellules susceptibles d'être utilisées en thérapie cellulaire, ou en pharmacologie expérimentale, notamment dans le cadre du criblage de médicaments.

L'invention concerne également l'utilisation d'inhibiteurs de LO tels que définis ci-dessus, pour la mise en oeuvre de procédés de culture *in vitro* de cellules en vue de l'obtention de tissus, tels que de la peau ou du cartilage, lesdits tissus étant susceptibles d'être utilisés en tant que greffons ou implants en thérapie tissulaire, ou en pharmacologie expérimentale, notamment dans le cadre du criblage de médicaments.

L'invention a également pour objet tout milieu de culture cellulaire caractérisé en ce qu'il contient un ou plusieurs inhibiteurs de LO tels que définis ci-dessus.

L'invention concerne également un procédé de culture *in vitro* de cellules telles que définies ci-dessus, au cours duquel le phénotype desdites cellules est maintenue à un stade identique à celui ou se trouvaient initialement lesdites cellules lors de leur mise en culture, ledit procédé comprenant la mise en culture desdites cellules dans un milieu approprié contenant un ou plusieurs inhibiteurs de LO tels que définis ci-dessus.

L'invention a plus particulièrement pour objet un procédé de maintien constant du phénotype de cellules différenciées en culture à un stade proche ou identique à celui où se trouvaient lesdites cellules lors de leur mise en culture, caractérisé en ce qu'il comprend la mise en culture desdites cellules dans un milieu approprié contenant un ou plusieurs inhibiteurs de LO tels que définis ci-dessus.

L'invention a également pour objet un procédé de préparation de cellules différenciées au phénotype identique, ou d'implants cellulaires constitués de telles cellules, caractérisé en ce qu'il comprend :
- la mise en oeuvre d'un procédé de culture *in vitro* de cellules tel que défini ci-dessus,
- le cas échéant, une ou plusieurs étapes de lavage des cellules en culture à l'aide d'une solution tampon appropriée, afin d'éliminer en tout ou partie le ou les inhibiteurs de LO utilisés,
- le cas échéant une étape de digestion enzymatique de la matière extracellulaire susceptible de s'être formée, à l'aide d'enzymes appropriées,
- le cas échéant une étape de récupération des cellules mises en culture.

L'invention concerne également un procédé de préparation *in vitro* d'une matrice cellulaire telle que définie ci-dessus, susceptible de pouvoir être utilisée en tant que tissus ou implants tissulaires, constitués de cellules différenciées, caractérisé en ce qu'il comprend :
- la mise en oeuvre d'un procédé de culture *in vitro* de cellules tel que défini ci-dessus, jusqu'à la formation d'une matrice cellulaire telle que définie ci-dessus, suffisante pour constituer une trame tissulaire,
- le cas échéant, une ou plusieurs étapes de lavage de la matrice cellulaire en culture à l'aide d'une solution tampon appropriée, afin d'éliminer en tout ou partie le ou les inhibiteurs de LO utilisés,
- le cas échéant une étape de récupération de la matrice cellulaire telle que définie ci-dessus.

L'invention a plus particulièrement pour objet un procédé de préparation *in vitro* de tissus ou implants tissulaires tel que défini ci-dessus, appliqué à la néosynthèse d'implants de cartilage lorsque ledit procédé est effectué à partir de chondrocytes, ou à la préparation de substitut cutanés lorsque ledit procédé est effectué à partir de fibroblastes et/ou de kératinocytes.

L'invention concerne également un procédé de criblage de molécules d'intérêt pharmacologique, notamment de médicaments, caractérisé en ce qu'il comprend :
- la mise en oeuvre d'un procédé de culture *in vitro* de cellules tel que défini ci-dessus, le cas échéant jusqu'à la formation d'une matrice cellulaire suffisante pour constituer une trame tissulaire,
- le cas échéant, une ou plusieurs étapes de lavage de la matrice cellulaire en culture à l'aide d'une solution tampon appropriée, afin d'éliminer en tout ou partie le ou les inhibiteurs de LO utilisés,
- une étape de mise en présence de la molécule testée avec les cellules ou tissus obtenus lors des étapes précédentes,
- la détection de l'éventuel effet de ladite molécule sur les cellules ou les tissus susmentionnés.

L'invention a également pour objet les cellules différenciées au phénotype maintenu, ou implants cellulaires constitués de telles cellules, tels que définis ci-dessus, caractérisés en ce qu'ils contiennent des inhibiteurs de LO tels que définis ci-dessus, le cas échéant à l'état de traces.

L'invention concerne également les matrices cellulaires ou tissus ou implants tissulaires à base de cellules telles que définies ci-dessus, caractérisés en ce qu'ils contiennent des inhibiteurs de LO tels que définis ci-dessus, le cas échéant à l'état de traces.

L'invention a plus particulièrement pour objet les cellules ou implants cellulaires, ou matrices cellulaires ou tissus ou implants tissulaires susmentionnés, tels qu'obtenus par mise en oeuvre d'un procédé tel que défini ci-dessus.

L'invention concerne également les implants de cartilage comprenant une matrice extracellulaire essentiellement exempte de collagènes normalement absents du cartilage sain (à savoir les collagènes I et III), et comprenant la totalité des collagènes spécifiques du cartilage (à savoir les collagènes II, IX, XI, et le cas échéant X).

L'invention a plus particulièrement pour objet les implants de cartilage susmentionnés, caractérisés en ce qu'ils contiennent des inhibiteurs de LO tels que définis ci-dessus, le cas échéant à l'état de traces.

L'invention a plus particulièrement pour objet encore les implants de cartilage susmentionnés, tels qu'obtenus par mise en oeuvre d'un procédé tel que défini ci-dessus.

L'invention concerne également les implants de chondrocytes, dont le phénotype initial, de type cartilage, a été maintenu pendant la phase préliminaire de multiplication cellulaire *in vitro.*

L'invention a également pour objet les implants tissulaires susmentionnés correspondant à des implants de peau reconstituée, ou substitut cutanés, tels qu'obtenus par mise en oeuvre d'un procédé selon la revendication 11 effectué à partir de fibroblastes et/ou de kératinocytes.

L'invention sera davantage détaillée à l'aide de la description qui suit des utilisations possibles des inhibiteurs de LO.

La fabrication d'implants tissulaires en génie tissulaire en général doit surmonter un obstacle majeur : éviter de produire un tissu anormal présentant des propriétés physico-chimiques incompatibles avec l'usage envisagé. La culture des cellules in vitro en vue de leur multiplication et de leur production d'une matrice extracellulaire s'accompagne malheureusement d'une altération de leur phénotype biochimique qui est plus ou moins profond suivant le type de culture de cellules.

La culture de chondrocytes a été jusqu'alors, de ce point de vue, toujours accompagnée de cette altération phénotypique. Les solutions proposées jusqu'alors pour les cultures de chondrocytes ont consisté à ajouter (ou retrancher parfois) des facteurs solubles à la culture (hormones, vitamines, facteurs cellulaires essentiellement), que celle ci soit effectuée sur support plastique plan ou dans un support en trois dimensions de diverses natures (éponges de collagènes plus ou moins réticulées associés à divers constituants, céramiques etc.).

La production de peau reconstruite est fondée sur l'interaction de fibroblastes et de kératinocytes placés dans des conditions particulières. Les interventions permettant d'améliorer cette production sont limitées et mal définies, souvent sur une base empirique. Plusieurs nutriments s'avèrent ainsi indispensables et ont été ajoutés aux conditions de culture. Le traitement au βAPN présente la singularité de s'adresser aux deux types cellulaires.

La méthode de la présente invention s'applique à tout type de culture en genie tissulaire. Elle consiste à agir sur l'état physique de la matrice extracellulaire synthétisée par les cellules en culture afin de moduler les signaux que celle ci envoie aux cellules, via divers récepteurs cellulaires, et ainsi de réguler l'état de différenciation des cellules isolées à partir de biopsies.

L'inhibition de la LO (notamment par le βAPN) a été jusqu'alors utilisée dans le cadre de procédés de production de collagènes, pour inhiber la réticulation des collagènes sécrétés par les cellules et améliorer ainsi les conditions de leur solubilisation. Un tel inhibiteur est, pour cette raison, classiquement reconnu pour altérer la matrice extracellulaire et n'a été jusqu'alors utilisé que dans trois circonstances :
- la production de collagènes non réticulés facilement extractibles,
- l'étude fondamentale du mécanisme d'action de la LO,
- contribuer à inhiber les pontages excessifs des collagènes dans certaines pathologies fibrosantes.

En dehors de ces trois domaines, l'inhibition de l'activité LO est apparue jusqu'alors comme une gêne, non comme un avantage.

Les principaux avantages de la présente invention sont les suivants.

Le βAPN, l'inhibiteur type de l'activité LO, est un amino-nitrile. Ce composé, très bon marché, n'a jusqu'alors jamais été utilisé pour maintenir le phénotype des cellules en culture. Non toxique aux doses employées pour les cellules en culture, son action peut néanmoins atteindre d'autres cellules au sein d'un organisme : il ne peut à ce titre être utilisé sans risques à des fins thérapeutiques, nécessairement ciblées. Son élimination des cultures avant leur utilisation comme implant est aisée par lavages successifs pour épuiser la culture en βAPN. Les autres inhibiteurs spécifiques de l'activité LO pourraient utilement être comparés quant à leur niveau de tolérance *in vivo* dans un tel modèle.

L'efficacité du βAPN à la concentration classique de 50 µg/ml a été totale après 2 semaines de culture où, en son absence, la dédifférenciation des chondrocytes était devenue maximale. Cette concentration est utilisable pour le traitement des peaux reconstruites.

L'arrêt du traitement par le βAPN permet alors de restaurer l'activité des LO qui sont sécrétées en continu (voire plus exprimées en présence de βAPN) par les cellules et présentent une durée d'activité limitée dans le temps. Bien que l'inhibition des LO par le βAPN soit irréversible, l'élimination du βAPN, par lavages et arrêt du traitement, permet de restaurer in *vitro* au moment choisi l'activité de cette enzyme. La réticulation souhaitée des fibrilles de collagènes et d'élastine, substrats des LOs, peut ainsi être programmée.

La méthode de la présente invention s'applique à tout type de culture en genie tissulaire, c'est à dire :
- tout type de support : le plus simple comme le support plastique des boîtes de culture, ou le plus sophistiqué comme certains modèles de culture en trois dimensions.
- tout type de cellules en vue d'implants tissulaires divers (cartilage, os, peau, cornée, vaisseaux sanguins, etc). Le traitement décrit peut être utilisé pour deux types d'implantations :
   a) suspension de chondrocytes multipliés in vitro. De plus, le traitement préalable de la culture par le βAPN améliore le rendement d'isolement des cellules viables par trypsination.
   b) matrice cartilagineuse riche en chondrocytes et de viscosité définie.

Dans le cas de cartilages articulaires lésés ou nécrosés localement (hanche en particulier), il peut être souhaitable de recourir à des injections d'une matrice riche en chondrocytes non dédifférenciés. La fluidité du tapis cellulaire est fonction de la durée de la culture et peut donc être aisément choisie par le chirurgien en fonction des besoins. Cet avantage est bien sûr généralisable à toute autre situation rencontrée en génie tissulaire.

Les tapis cellulaires ainsi obtenus après un temps donné de contact avec un inhibiteur de LO peuvent également servir de support d'étude en pharmaco - toxicologie.

### 1. Descriptif détaillé

### 1.1.) Matériel et Méthodes

### Modèle de culture de chondrocytes :

a) Le modèle expérimental de culture de chondrocytes est celui de la culture sur plastique en deux dimensions de chondrocytes embryonnaires de poulet. L'état embryonnaire des cellules permet un haut niveau de synthèse des collagènes, contrairement aux chondrocytes adultes. Le sternum de poulet d'où sont issus les chondrocytes permet d'obtenir deux sous populations pures de chondrocytes capables ou non de s'hypertrophier ultérieurement (respectivement, les chondrocytes de la partie craniale ou caudale du sternum). Les biopsies de cartilage bovin ou humain ne permettent pas cette distinction. Les résultats qui sont décrits distinguent toujours la sous-population des chondrocytes étudiés.

Les cultures avec antibiotiques L-glutamine 4 mM, et 25 µg/ml ascorbate (facteur nécessaire à la stabilité des unités en triple hélice de collagènes sécrétés) sont poursuivies pendant 2 semaines en présence ou en absence de 10% sérum, remplacé éventuellement par l'insuline 100 ng/ml, et de 50 µg/ml βAPN. L'absence de sérum nécessite l'adjonction d'agents protecteurs (1 mM cystéine et 1 mM pyruvate). Un jour avant prélèvements, 10 µCi de 14C Proline sont ajoutés aux cultures dont les tapis ou les milieux seront ensuite pepsinés (0.2 mg pepsine /ml / pH 4/ 24 hrs) et soumis à l'analyse électrophorétique (SDS PAGE en gradient de 4,5 à 15% d'acrylamide) puis fluorographique. Les fluorogrames révèleront alors les seules bandes protéiniques pepsino-résistantes, essentiellement les collagènes et des lipocalines (protéines de transport lipidique). La position connue de ces bandes permet de signer la dédifférenciation des cellules par l'apparition de la chaine α2 I du collagène I associée à la baisse de synthèse des collagènes de type cartilage.

L'activité Lysyl oxydase a été dosée avec 300.000 dpm d'élastine marquée sur la Lysine, substrat de l'enzyme (Shackelton et Hulmes Anal. Biochem.(1990) 185 : 359-362) . La détermination de la présence de Lysyl oxydases dans les cultures de chondrocytes est effectuée selon les méthodes classiques d'immuno-décoration des supports où ont été transférées les bandes séparées des protéines extraites à partir des milieux de culture ou des tapis cellulaires correspondants.
b) Les cultures de chondrocytes humains normaux proviennent de cartilages prélevés lors d'interventions de prothèses de hanche ou de ligamentoplastie, puis soumis à une digestion enzymatique. Ces biopsies de cartilage sont considérées comme res nullus.

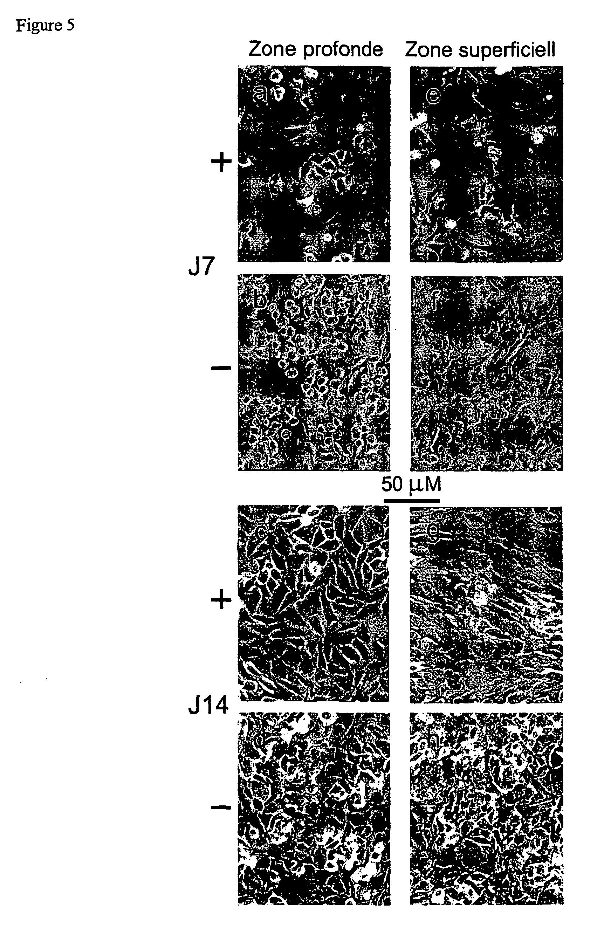

Les chondrocytes extraits du bourelet cotyloïdien ont été utilisés pour la culture en deux dimensions sur plastique en présence de BAPN: ont été comparées les cultures obtenues après digestion enzymatique des cartilage en présence ou en absence de hyaluronidase (figures 4 et 5).

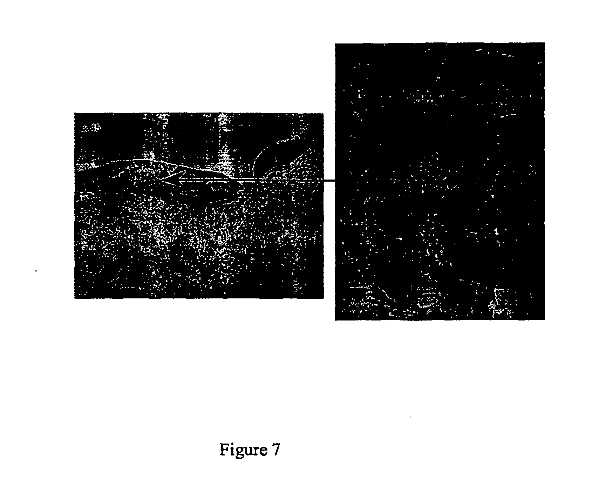

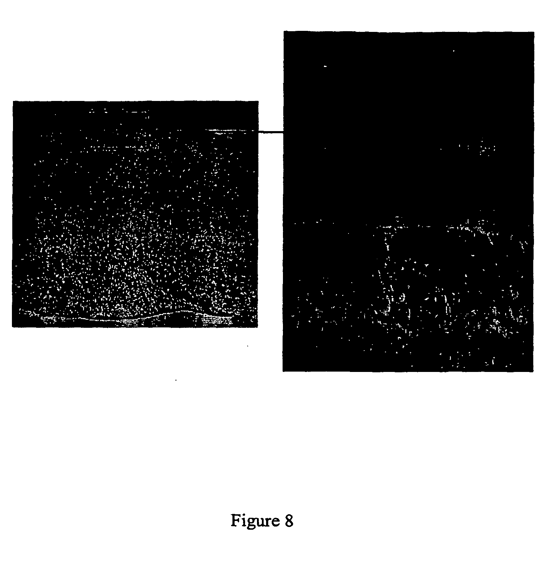

Les chondrocytes obtenus après ligamentoplastie ont été utilisés pour la culture en trois dimensions en éponge de collagène-GAG-chitosan (Collombel et al. 1987) * en présence ou en absence de BAPN (figures 7 et 8).

* Collombel C., Damour O., Gagneu C., Poinsignon F., Echinard C., Marichy J. : Peau artificielle et son procédé de fabrication. Brevet d'invention français n° 87-08752 (15 juin 1987), brevet d'invention européen n° 88- 420194.8 (14 juin 1988).

### Modèle de culture de peau reconstruite :

Le procédé permettant la formation de la peau reconstruite est celui décrit dans le brevet français 87 08252 du 15 juin 1987.

Le support du modèle de peau reconstruite est constitué d'un substrat dermique (SD) à base de collagène-glycosaminoglycanne réticulé par du chitosane selon la technique publiée par Duplan-Perrat et al (J Invest Dermatol 2000 114:365). Des fibroblastes sont ensemencés à l'intérieur du SD pour obtenir un derme équivalent. La peau reconstruite (PR) est obtenue par ensemencement des kératinocytes après 15 jours (J15) de culture en derme équivalent. Les fibroblastes dermiques sont obtenus à partir d'explants de prépuces humains après séparation dermo-épidermique et cultivés en monocouche en milieu DMEM (Sigma) supplémenté par 10% de sérum de veau foetal, de L-glutamine 4 mM, d'EGF (Austral Biologic) à 10 ng/ml, de pénicilline à 100 UI/ml, de gentamicine à 100 µg/ml et d'amphotéricine B à 1 µg/ml. Les kératinocytes sont issus d'un prélèvement de biopsie de peau humaine après séparation dermo-épidermique par trypsination. Les cellules sont ensemencées sur des couches nourricières (fibroblastes irradiés) et cultivées en en DMEM et HAM F12 à 30% supplémenté en sérum de veau foetal à 10%, d'EGF à 10 ng/ml, d'hydrocortisone à 1,6 ng/ml, d'umuline à 0,12 UI/ml, de cholératoxine à 0,1 nM, de triiodothyronine à 0,2 µM, d'adénine à 24 µg/ml, de L glutamine à 4 mM, de pénicilline à 100 UI/ml, de gentamicine à 100 µg/ml et d'amphotéricine B à 1 µg/ml

Un mélange à base de collagène bovin de type I et III (72%), de glycosaminoglycanne (8%) réticulé par du chitosane (20%) est coulé dans des plaques 6 puits. Les substrats dermiques ainsi obtenus sont ensuite congelés à -80°C et lyophilisés. Les fibroblastes sont ensemencés dans le substrat dermique réhydraté et stérilisé à raison de 250 000 cellules par cm². Des kératinocytes sont ensemencés après 15 jours (J15) avec une densité de 200 000 à 250 000 cellules/ cm². Les peaux reconstruites sont cultivées 8 jours de façon immergée, dans le milieu décrit précédemment supplémenté de 50µg/ml de vitamine C. Ensuite les peaux reconstruites sont élevées à l'interface air liquide en utilisant un milieu de culture simplifié à base de DMEM, supplémenté en sérum de veau foetal, d'EGF, d'hydrocortisone, d'umuline, de L-glutamine, de pénicilline, de gentamicine, et d'amphotéricine B, et d'acide ascorbique, aux concentrations décrites ci-dessus. Les peaux reconstruites sont cultivées jusqu'à J60. Des prélèvements sont réalisés à J30 et J60 pour les différentes analyses.

### Utilisation de l'inhibiteur de Lysyl oxydases :

Le βAPN (50µg/ml) est ajouté dès l'ensemencement, au milieu de culture renouvelé tous les 2 jours et fourni aux cultures de chondrocytes comme à celles de fibroblastes puis de kératinocytes lors de la formation de la peau reconstruite. La concentration de 50µg/ml n'est pas toxique pour les cellules et n'affecte pas sensiblement la multiplication cellulaire. Des concentrations plus élevées de 200µg/ml s'avèrent avoir peu d'effet sur la viabilité et la multiplication des fibroblastes et des kératinocytes en cultures isolées.

### 1.2.) Résultats

Des anticorps spécifiques de diverses enzymes de la famille lysyl oxydase ont permis d'identifier pour la première fois ces différentes isoformes dans les chondrocytes, tant par immuno-décoration après séparation électrophorétique que par immuno-marquage de tapis cellulaires observé au microscope à fluorescence. Une activité lysyl oxydase spécifique a aussi, pour la première fois, été démontrée dans les milieux de culture des chondrocytes (tableau I).

Les anticorps anti LO ont aussi permis de détecter ces isoformes dans la peau reconstruite. Des résultats ont mis en évidence une forte expression des LOX (figure 4), LOXL1 et LOXL2 au niveau du derme, de l'épiderme et de la jonction dermo-épidermique. L'observation d'une expression de ces isoformes de LO dans des kératinocytes est tout à fait originale à ce niveau, où il n'y a synthèse ni de collagène ni d'élastine.

### Régulation du phénotype "cartilage" des chondrocytes :

En présence de sérum, avec lequel les taux de multiplication et de synthèse protéiques sont optimaux, les chondrocytes étudiés se dédifférencient nettement après la première semaine de culture, en accord avec les données de la littérature. L'adjonction de βAPN pendant toute la culture supprime toute dédifférenciation, comme l'atteste l'absence de la chaine collagénique α2I sur les fluorogrammes. De même, le βAPN supprime également les altérations morphologiques observées par ailleurs dès les premiers jours dans les cultures non traitées où sont bien visibles des cellules de forme fibroblastique.

En l'absence de sérum, les chondrocytes adhèrent bien sur le support plastique mais s'étalent très difficilement, voire pas du tout pour les chondrocytes caudaux. La présence d'un anabolisant des chondrocytes, comme l'insuline, augmentent quelque peu les taux de synthèse, n'induisent pas de dédifférenciation biochimique avec ou sans βAPN, mais laissent apparaître une altération morphologique en l'absence de βAPN (Fig. 1).

La présence de sérum dans le milieu de culture est donc néanmoins souhaitable pour obtenir un taux élevé de multiplication et de synthèses, le βAPN permettant de supprimer l'altération du phénotype biochimique et morphologique.

Les cultures de chondrocytes ainsi traitées mais étudiées entre le 2ème et le 3ème jour ont montré la présence de collagènes normaux aussi bien dans le tapis cellulaire que dans le milieu de culture. Néanmoins, le βAPN a permis l'apparition normale de collagène X par les chondrocytes hypertrophiques : la baisse de cette synthèse, en l'absence de βAPN, est connue pour précéder la synthèse de collagène I, marqueur de dédifférenciation (fig 2). Ces collagènes, tous de type cartilage, sembleraient donc bien acquérir, sous l'effet de pontages dépendants de LO, une structure qui progressivement contribuerait à l'envoie de signaux aux chondrocytes, à l'origine de l'altération du phénotype biochimique en culture in vitro.

Il restait à prouver l'origine de cette inhibition de dédifférenciation par le βAPN. Les cultures en présence de βAPN ne modifient en rien la synthèse et la sécrétion de ces LO (Fig 3). Le βAPN n'agit donc bien que sur l'activité amine oxydase de l'enzyme, et non sur sa sécrétion.

### Application du modèle aux cellules de cartilage humain.

Le mode d'extraction des cellules par digestion enzymatique du cartilage permet de mettre en évidence des sous populations différentes de chondrocytes au sein d'une même biopsie de cartilage. C'est le cas des chondrocytes superficiels et profonds du cotyle du cartilage fémoral après ajout de hyaluronidase dans le milieu d'extraction. A l'ensemencement, ces deux populations de cellules produisent bien du collagène II, mais pas de collagène I. Seuls, les chondrocytes profonds vont ensuite être capable en présence de βAPN de conserver leur morphologie et leur phénotype pendant les deux semaines suivantes où elles se multiplient en produisant une matrice extracellulaire de type cartilagineux, dépourvue de collagène I.

Il est donc nécessaire d'utiliser aux fins de Génie tissulaire des zones de cartilage dont les populations de chondrocytes sensibles au βAPN ont été correctement isolées.

Néanmoins, des chondrocytes humains dédifférenciés aux termes du temps de culture nécessaire à leur multiplication en culture sur plastique, peuvent être, avec profit, mis en culture tridimensionnelle 3D en présence de βAPN. La mise en culture 3D pouvant ne pas suffire, à elle seule, à assurer un retour au phénotype cartilage perdu pendant la culture en 2 dimensions en présence de sérum, tel que cela a été décrit pour des cultures 3D en agarose. Effectivement, l'immunofluorescence en triple marquage des collagène I, collagène II et aggrécane sur des coupes obtenues à partir de cultures tridimensionnelles de chondrocytes en éponge a permis de mettre en évidence l'effet favorable du BAPN sur la préservation du phénotype cartilage des chondrocytes après trois mois de culture :
une extension de la zone de dépôt de collagène II dans l'éponge et une nette augmentation de l'intensité de marquage du collagène II et de l'aggrécane (marqueurs du cartilage) associée à une forte diminution de celle du collagène I dues au βAPN.

Il est nécessaire de s'assurer de l'efficacité du traitement destiné à éliminer le βAPN de la culture de cellules avant son utilisation comme implant tissulaire autologue. Cette opération répond à deux exigences :
1°- rétablir une activité lysyl oxydase normale au sein du cartilage implanté en cours de réparation.
2°- éliminer le risque , hypothétique mais non démontré, d'un éventuel effet préjudiciable du βAPN sur les cellules du patient.

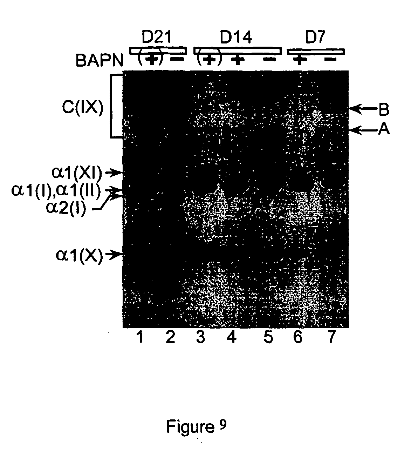

Les modifications, observées pour la première fois, sous l'effet de l'inhibition par le βAPN de l'activité lysyl oxydase, dans les pontages entre chaînes de collagène IX se sont avérées très précoces (précédant l'apparition de collagène I marqueur de dédifférenciation) et réversibles. (au plus tard dans les 6 jours qui succèdent à l'élimination du βAPN)(figure 9). Nous avons prouvé ainsi que des lavages répétés du tapis cellulaire par du PBS précédant l'usage de milieu de culture sans βAPN s'avèrent suffisants pour éliminer toute trace de βAPN actif.

Il a été par ailleurs vérifié qu'un ensemencement à haute densité retarde bien l'apparition de la dédifférenciation.en culture 2D.

### Régulation de la formation de la peau reconstruite :

### Etude préliminaire du βAPN sur les fibroblastes et les kératinocytes cultivés en monocouche

La numération cellulaire montre que le βAPN ne présente un effet inhibiteur sur la prolifération des fibroblastes et des kératinocytes que pour des doses relativement fortes. En effet, le nombre de ces cellules est diminué d'environ 50% par rapport au témoin lorsqu'elles sont cultivées en présence de 600 µg/ml de βAPN. Cependant, à la concentration de 200 µg/ml, la prolifération des fibroblastes est légèrement augmentée, alors que celle des kératinocytes est diminuée d'environ 10%. En revanche, aucun effet toxique du βAPN n'est observé sur les fibroblastes et les kératinocytes à confluence jusqu'à 800 µg/ml. Les résultats du test de viabilité au MTT confirment ceux de la numération cellulaire. En conclusion, ces résultats indiquent que le βAPN, à des concentrations élevées (supérieures à 500 µg/ml), présente un effet inhibiteur sur la prolifération des fibroblastes et des kératinocytes. Il faut souligner que le βAPN à 200 µg/ml peut éventuellement engendrer une augmentation de l'activité cellulaire (prolifération et viabilité) sur les fibroblastes ensemencés à faible densité, et sur les fibroblastes et les kératinocytes ensemencés à forte densité.

### Peaux reconstruites (PR)

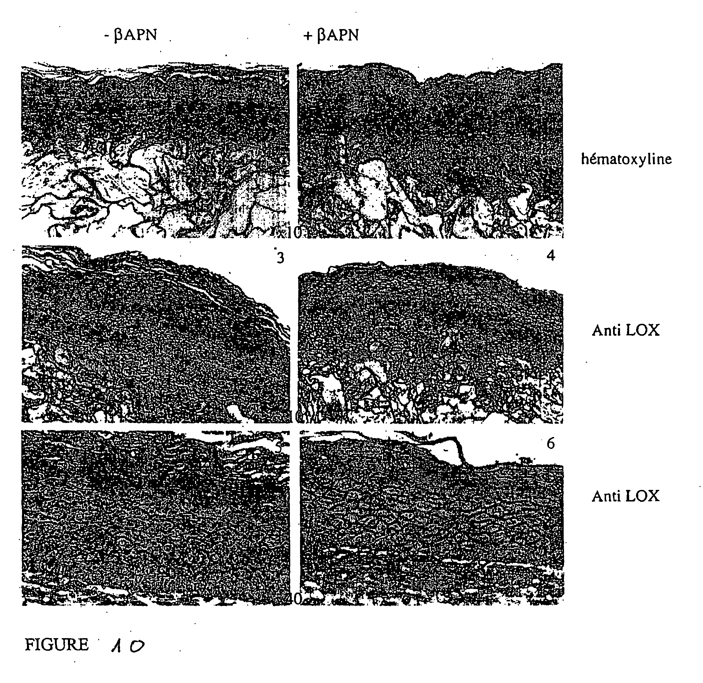

Un effet important du βAPN pour les greffes serait la réduction possible de la rétractation induite par la réticulation des collagènes (figure 10).

La surface des peaux reconstruites immergées traitées au βAPN est supérieure à celle des PR immergées non traitées, suggérant une atténuation du phénomène de rétractation. En effet, si l'on considère les résultats obtenus avant l'élévation à l'interface air-liquide, les peaux témoin non traitées présentent une diminution de surface de 40% par rapport à leur état original, contre 17% seulement pour les peaux traitées par 500 µg/ml de βAPN.

A 35 jours de culture, la surface des peaux reconstruites témoin ou traitées par 50 µg/ml de βAPN est de 50% par rapport à la surface de départ, tandis que les peaux reconstruites traitées à 200 et 500 µg/ml de β-APN ne présentent qu'une diminution de surface de 40%. L'effet du βAPN observé sur la surface des peaux reconstruites après deux semaines de culture à l'interface air-liquide n'est cette fois plus dose-dépendant. On note bien une surface significativement supérieure des PR traitées par 200 ou 500 µg/ml de βAPN, par rapport à celle du témoin. Les PR traitées par 50 µg/ml de βAPN ne montrent aucune différence significative de leur surface par rapport au témoin non traité.

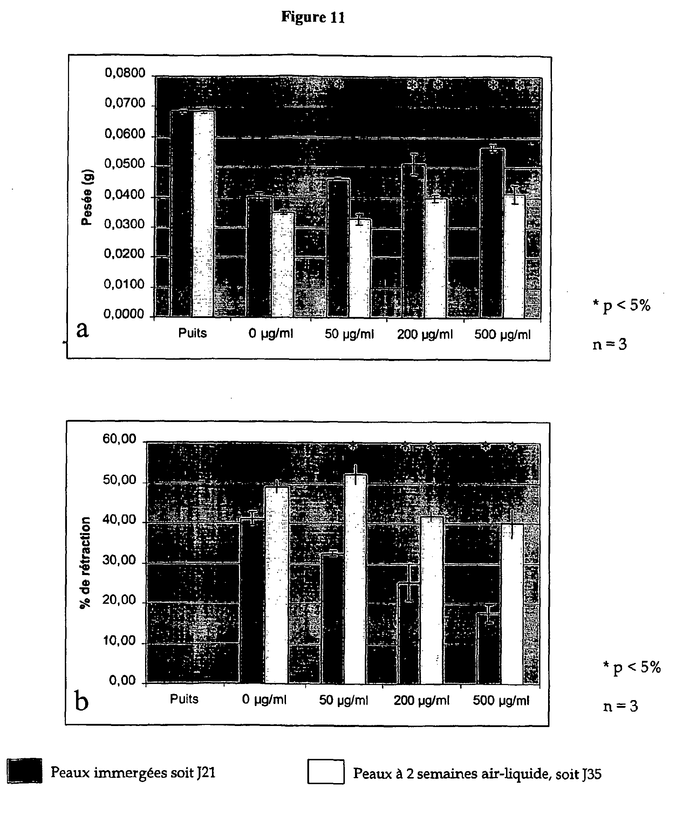

L'histologie des PR (figure 11) ne semble pas fondamentalement changée par le traitement au βAPN à 50 µg/ml ou 200 µg/ml. Les PR traitées apparaissent constituées d'un derme présentant une matrice extracellulaire (MEC) riche, et d'un épiderme présentant les couches basales, spineuses, granuleuses et cornées. Même si les PR formées sont plus fragiles et l'épiderme plus fin, la structure générale classique de l'épiderme est conservée. La colonisation initiale de l'éponge par les fibroblastes est favorisée par la présence de βAPN, ce qui se traduit par une synthèse accrue de MEC.

Dans la peau normale et dans notre modèle de peau reconstruite, les kératinocytes de la couche basale représentent les cellules souches. L'évolution de leur phénotype concomitant et leur migration vers les couches externes de l'épiderme vont permettre la formation de la couche cornée. Cette couche cornée est indispensable, car elle assure la fonction de barrière de la peau. Sa formation doit cependant être contrôlée afin de permettre la synthèse d'échantillons de peau suffisamment important. Même si l'apport temporaire de βAPN semble ralentir l'évolution du phénotype initial des kératinocytes, l'organisation générale en couches basales, granuleuse, spineuse et cornée est maintenue.

Les analyses immunohistologiques et ultrastructurales révèlent des modifications significatives des PR traitées. Les constituants structurants de la MEC que sont les collagènes fibrillaires voient leur organisation en fibres modifiées. Les molécules de collagène sécrétées sont déposées sous forme de fibres de diamètres irréguliers, présentant des fusions entre fibres adjacentes. Nous avons enfin montré par hybridation in situ que le traitement par 50 µg/ml de βAPN se traduisait par une légère augmentation de la détection des gènes de LOX et LOXL, à la fois dans le derme et l'épiderme.

### Légendes des figures

**Fig 1** : Effet de l'activité lysyl oxydase sur la dédifférenciation de chondrocytes après 2 semaines de culture en deux dimensions sur plastique

4 millions de chondrocytes de la partie craniale du sternum de poulet embryonnaire ont été ensemencés par boite de Petri de 35 mm de diamètre (P35) en milieu DMEM avec ou sans serum10% (remplacé par insuline 100ng/ml) avec ou sans βAPN (50 µg/ml). Les milieux de culture avec 25 µg/ml d'ascorbate sont changés tous les deux jours. Les collagènes neosynthétisés marqués à la Proline 14C pendant les dernières 24 heures sont isolés après pepsination classique des milieux de culture conditionnés puis soumis à SDS PAGE, sans conditions réductrices, et fluorographie.

Observations :
- en présence de serum, fortement anabolisant, la dédifférenciation biochimique des chondrocytes (collagène I sécrété, arrêt de synthèse de collagène X) est totalement inhibée sous l'action du βAPN qui augmente la proportion de collagène X spécifique de ces chondrocytes hypertrophiques. De même, le βAPN préserve l'hypertrophie et la morphologie non étalée caractéristiques de ces chondriomes.
- en l'absence de serum, les chondrocytes adhèrent au plastique des boites mais ne s'étalent pas. Après 15 jours de culture, l'absence de βAPN ne génère que la seule altération morphologique, mais la croissance cellulaire reste dans l'ensemble ralentie malgré la présence d'insuline.

**Fig 2** : Effet de l'activité lysyl oxydase sur la dédifférenciation de chondrocytes après 3 jours de culture, en présence de serum, en deux dimensions sur plastique

L'étude fluorographique décrite pour la figure 1 a portée ici sur les tapis et milieux de culture pepsinés

Observations :
Seule la présence de βAPN permet l'expression normale de collagène X (bandes inférieures des fluorogrammes) par les chondrocytes hypertrophiques (craniaux) identifié dans les milieux et les tapis cellulaires. L'inhibition de la synthèse de collagène X est connue pour précéder l'apparition de collagène I par les chondrocytes en cours de dédifférenciation, qui s'initie donc aux premiers jours de culture alors que les collagènes anormaux n'ont pas encore été synthétisés.

**Fig 3** **:** Immunodécoration par anticorps anti LOX, LOXL1, d'extraits de tapis cellulaires et de milieu de culture de chondrocytes soumis à SDS PAGE puis transfert sur membrane de nitro cellulose.

Le jour qui précède les prélèvements, le tapis cellulaire est lavé trois fois par du milieu sans serum. Le milieu est alors changé pour 24 heures par du milieu complet sans serum.

Au 6° ou 14° jour de culture les milieux sont prélevés et précipités classiquement par TCA 10% final / 4°C / 30 mn. Les culots de centrifugation sont rincés à l'acétone puis solubilisés dans un 50 µl de tampon d'électrophorèse. Les tapis cellulaires rincés sont lysées 2h / 4°C (300 µl/P35) dans le mélange urée 8M, Nonidet 0,5%, phosphate de sodium disodique 16 mM pH 8 et inhibiteurs de protéases. La concentration d'urée est amenée à 4M avant centrifugation de la suspension. Les surnageant sont précipité par TCA puis traités comme pour les milieux correspondants.

Après séparation par SDS PAGE et transfert par Western Blot, les bandes proteiniques sont analysées par immunodécoration à l'aide d'anticorps anti-LOX et anti-LOXL1 polyclonaux de lapin, puis d'un 2° anticorps anti lapin marqué à la phosphatase alcaline avant révélation au réactif NBT/BCIP (Roche Diagnostic GmBH, Manheim, Germany).

Observations :
1) La LOX et la LOXL1 sont exprimées par les chondrocytes cultivés dans les conditions décrites pour le dosage d'activité enzymatique. Les Lysyl oxydases accumulées dans les tapis après 1 à 2 semaines peuvent être mises en évidence, à l'inverse des milieux de culture de seulement 24 hrs.
2) La présence de βAPN ne modifie pas l'expression des molécules de Lysyl oxydases, seule l'activité enzymatique est inhibée comme le montre le tableau I.

**Fig 4** : Identification par fluorographie des collagènes radiomarqués synthétisés par les chondrocytes de cartilages cotyloïdiens en culture 2D sur plastique. Effets de la hyaluronidase ajoutée au milieu de digestion enzymatique des cartilages

Les radiomarquages de 24 hrs à la proline 14C ont été effectués au 2°, 7° et 14° jour de i culture en présence de sérum et βAPN (D2, D7 et D14 respectivement). Les extraits pepsinés de milieu de culture et de tapis cellulaire ont été soumis à électrophorèse puis fluorographie.

Les zones superficielle (n° pairs) et profonde (n° impairs) du cartilage cotyloïdien ont été séparées avant d'être soumises à la digestion enzymatique en présence ou non de hyaluronidase.

Observations :
b) les deux sous-populations de chondrocytes, profonde et superficielle, présentent un phénotype cartilage au 2° jour de culture, où elles ne synthétisent pas de collagène I, dans le tapis cellulaire comme dans le milieu.
c) En présence de hyaluronidase pendant la digestion préalable du cartilage: la zone profonde continue à ne pas synthétiser de collagène I, au 7° comme au 14° jour de culture.

Inversement, les cellules de la zone superficielle se dédifférencient en produisant du collagène I de façon croissante du 7° au 14° jour de culture. Du collagène I neosynthétisé est également présent dans le tapis cellulaire. Du collagène III est produit tardivement et en faible proportion au 14° jour dans le milieu de culture, mais est très peu retenu dans le tapis cellulaire, à l'inverse du collagène II.
d) En l'absence de hyaluronidase pendant la digestion préalable du cartilage: les deux sous- populations de chondrocytes qui viennent d'être définies évoluent de façon similaire au cours des deux semaines de culture en se dédifférenciant.

Fig 5 : Morphologie des cellules de cartilage cotyloïdien en culture 2D sur plastique.

Les observations ont été effectuées aux 7° et 14° jours de culture (D7 et D14) en présence de sérum et βAPN sur les mêmes cultures utilisées pour les radiomarquages.

Digestions enzymatiques préalables des cartilages en présence (+) ou absence (-) de hyaluronidase.

Observations :
e) digestion en présence de hyaluronidase (+): au 7° jour de culture (a, e) : les cellules de la zone profonde présentent une morphologie caractéristique des chondrocytes , telle qu'elle peut être observée in situ. A l'opposé, les cellules de la zone superficielle, de type fibroblastoïde, présentent de nombreux et fins prolongements. Ces différences sont maintenues au cours des multiplications successives au moins jusqu'au 14° jour de culture (c, g).
f) digestion en absence de hyaluronidase (-): les cellules sont incluses dans une matrice extracellulaire plus abondante où les différences morphologiques entre les deux sous-types de cellules sont moins nettes (b, f, d et h).

**Fig 6** : Structure du cartilage humain normal

**Fig 7** : Chondrocytes humains normaux cultivés 1 mois en 3D sous agitation en présence de BAPN

Culture en 3 D dans des éponges de collagène-GAG-chitosan en présence de serum et βAPN de chondrocytes humains normaux. Les chondrocytes ont été préalablement multipliés, et dédifférenciés, en culture 2D sur plastique.

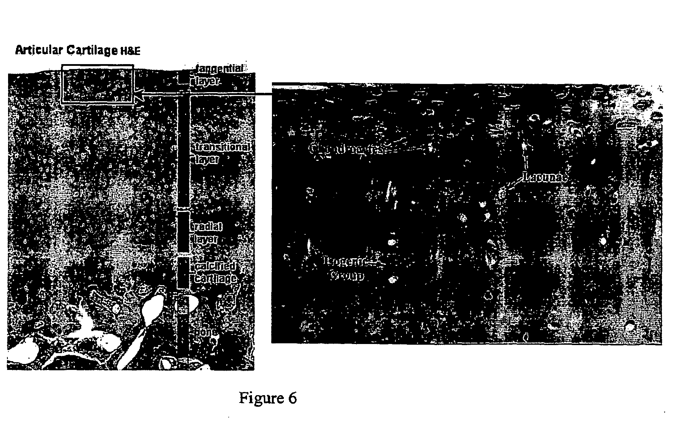

L'analyse histologique montre en surface la présence d'une zone dense épaisse de structure histologique très proche de celle du cartilage humain normal (figure 6). Les chondrocytes sont entourés d'une matrice extracellulaire importante qu'ils ont néosynthétisée et sont logés dans de petites lacunes. Plus profondément, on observe une colonisation de l'éponge avec une synthèse de matrice très pauvre.

**Fig 8** : Chondrocytes humains normaux cultivés 1 mois en 3D sous agitation sans βAPN

Les mêmes éponges de collagène-GAG-chitosan traitées ici sans βAPN présentent en surface une zone dense peu épaisse localisée sur une surface limitée de l'éponge. Plus profondément, la colonisation et la synthèse de matrice ne diffèrent pas des éponges traitées au βAPN.

En conclusion le traitement au βAPN semble favoriser le développement d'une structure proche de celle du cartilage humain normal en surface.

**Fig 9** : Réversibilité des effets du βAPN sur les pontages des chaines de collagène IX produit dans le tapis cellulaire de chondrocytes hypertrophiques de poulet embryonnaire. Etude fluorographique.

Des chondrocytes de la partie craniale de sternum ont été ensemencés à forte concentration (5 millions de cellules par boite de Petri P35) puis soumis à 24 hrs de radiomarquage à la proline 14C aux 7°, 14° et 24° jour de culture (respectivement D7, D14 et D21).

Le βAPN a été présent (+) ou absent (-) pendant toute la durée de culture, ou présent seulement pendant la première semaine ((+)).

Observations :
La présence de deux bandes principales de collagène IX varie en fonction de la présence ou non de βAPN pendant toute la culture : la bande A est reliée à l'absence de βAPN, alors que la bande B est observée essentiellement en présence de βAPN. La présence de βAPN limitée à la seule première semaine donne un profil électrophorétique après 2 et 3 semaines de culture qui est caractéristique d'une culture sans βAPN.

Dans tous les cas, la synthèse de collagène X augmente avec l'age de la culture, mais la synthèse de collagène 1 est ralentie par la forte densité d'ensemencement initiale (5 millions de cellules / P35 au lieu de 1,5).

Conclusions :
L'effet du βAPN sur les pontages du collagène IX est montré, il précède celui sur la dédifférenciation caractérisée par la synthèse de collagène I. Ces résultats montrent :
   b) la réversibilité des effets du βAPN.
   c) l'efficacité de l'élimination du βAPN de la culture cellulaire après lavages répétés des tapis cellulaires puis utilisation d'un milieu de culture sans βAPN.

**Fig 10****:** Analyse immunohistochimique de la peau reconstruite avec et sans βAPN
1 et 2 : coloration témoin à l'hématoxyline sans 1° anticorps.
3 à 6 : immunodétection de la LOX par un 2° anticorps marqué à la peroxydase en absence (1, 3, 5) ou en présence (2, 4, 6) de βAPN

Les colorations brun sombre marquent la présence de l'antigène LOX et sont absentes des coupes Témoin.

Après 35 jours de formation, la peau reconstruite est constituée d'un derme déposé sur l'éponge de collagène-glycosaminoglycanne-chitosane, et d'un épiderme formé par les kératinocytes.

Au niveau du derme, le traitement au βAPN permet une colonisation plus importante de l'éponge par les fibroblastes, ce qui se traduit par une synthèse accrue de matrice extracellulaire (1 et 2). L'expression de LOX apparaît plus intense.

Au niveau de l'épiderme, le stratification générale est retrouvée, mais le traitement au βAPN fait apparaître une ligne granuleuse plus intense. L'expression de LOX est également augmentée.

### Fig 11 : Effet du β-APN sur la rétraction de peaux reconstruites :

Le β-APN entraîne une diminution dose-dépendante et significative de la rétraction par rapport au témoin, lorsque les peaux sont encore en culture immergée (en gris). Après deux semaines à l'interface air-liquide (en blanc), la rétraction est toujours diminuée par rapport au témoin mais pour les concentrations 200 et 500 µg/ml seulement.

### TABLEAU I

Activités lysyl oxydase recueillie dans les tapis cellulaires de chondrocytes en culture sur plastique.

Deux populations de chondrocytes de sternum d'embryon de poulet (hypertrophiques dans la partie craniale, synthétisant du collagène X, et non hypertrophiques dans la partie caudale) Ensemencement: 4 millions cellules / Pétri 35mm (P35).

Culture + 25 µg/ml ascorbate, + 1 mM pyruvate, ± 10% FCS, ± 1 mM cys. 24 hrs avant la collecte milieu sans sérum (1,5 ml) le tapis cellulaire est lavé par le milieu sans sérum + cys. Des aliqotes milieu de culture après 24 hrs sont concentrés 10 fois puis soumis au dosage de l'activité Lysyl oxydase avec différents lots de substrat élastine tritiée.

| | dpm -βAPN | dpm + βAPN | activité / P 35 |
|---|---|---|---|
| **expérience 1**(substrat n°1) (culture - FCS, + cys) jour 3 | | | |
| | | | |
| chondrocytes caudaux | 930 | 674 | 1925 |
| (milieu de 15% P35) | 1002 | 718 | |
| | | | |
| **experience 2** (substrat n°2) (culture +FCS, - cys) jour 7 | | | |
| chondrocytes caudaux | 8158 | 5296 | 10232 |
| (milieu de 25% P35) | 8464 | 6210 | |
| | | | |
| chondrocytes craniaux | 10932 | 5800 | 4553 |
| (milieu de 100 % P35) | 10510 | 6536 | |
| | | | |
| LO aortique purifiée standard | 8544 | 4340 | / |
| | 8810 | 4762 | |
| jour 13 chondrocytes caudaux | 5016 | 4344 | 861 |
| (milieu de 75% P35) | 5322 | 4702 | |
| | | | |
| chondrocytes craniaux | 5038 | 4580 | 840 |
| (milieu de 100% P35) | 5840 | 4618 | |

### Conclusions :

Des activités lysyl oxydase (inhibées par le βAPN et dosées par la production d'eau tritiée) sont sécrétées dans les milieux de culture. L'activité spécifique recueillie dans les milieux est plus fort au 7° jour de culture qu'au 13° jour. La matrice extracellulaire étant plus riche en substrats collagèniques naturels de l'activité lysyl oxydase au 13° jour de culture, elle retiendrait donc l'enzyme dans le tapis cellulaire.

## Revendications

1. Utilisation d'inhibiteurs directs des lysyl oxydases (LO) choisis parmi :
les amines primaires réagissant avec le groupe carbonyle du site actif des LO, et plus particulièrement celles donnant, après liaison au carbonyle, un produit stabilisé par résonnance, telles que les amines primaires suivantes :
- l'éthylènediamine,
- les aminonitriles, tels que le β-aminopropionitrile (β-APN), ou la 2-nitroéthylamine,
- les haloamines, insaturées ou saturées, telles que la 2-bromo-éthylamine, la 2-chloroéthilamine, la 2-trifluoroéthylamine, la 3-bromopropylamine, les p- halobenzylamines, et les composés halogénés insaturés,
- la sélénohomocystéine lactone,
en tant qu'inhibiteurs de la dédifférenciation des cellules en culture *in vitro,* en vue du maintien constant du phénotype desdites cellules dans le cadre de la mise en oeuvre de procédé de culture *in vitro* de ces dernières, et ce pendant la totalité du temps de culture, pour la préparation de cellules différenciées dont le phénotype est identique, ou pour la préparation d'une matrice cellulaire constituée de cellules différenciées conservant le même phénotype et cultivées en présence desdits inhibiteurs, et du milieu extracellulaire secrété par lesdites cellules et liant ces dernières dans ladite matrice.

2. Utilisation du β-APN comme inhibiteur des LO selon la revendication 1 .

3. Utilisation d'inhibiteurs de LO selon l'une des revendications 1 à 2, pour la mise en oeuvre de procédés de culture *in vitro* de toutes cellules d'origine humaine ou animale, dans lesquels lesdites cellules sont maintenues à un phénotype de différenciation constant, et sont choisies notamment parmi les chondrocytes des cartilages, les cellules de la cornée, les cellules de la peau (tels que les fibroblastes de derme, et kératinocytes de l'épiderme), les cellules endothéliales des vaisseaux, les ostéoblastes des os, les hépatocytes, les cellules rénales, les cellules musculaires, les cellules souches ou pluripotentes.

4. Utilisation d'inhibiteurs de LO selon l'une des revendications 1 à 3, pour la mise en oeuvre de procédés de culture *in vitro* de cellules.

5. Utilisation d'inhibiteurs de LO selon l'une des revendications 1 à 3, pour la mise en oeuvre de procédés de culture *in vitro* de cellules en vue de l'obtention de tissus, tels que de la peau ou de cartilage .

6. Procédé de culture *in vitro* de cellules telles que définies dans la revendication 3, au cours duquel le phénotype desdites cellules est maintenu à un stade identique à celui ou se trouvaient initialement lesdites cellules lors de leur mise en culture, ledit procédé comprenant la mise en culture desdites cellules dans un milieu approprié contenant un ou plusieurs inhibiteurs de LO définis dans l'une des revendications 1 à 2.

7. Procédé de préparation de cellules, ou d'implants cellulaires, **caractérisé en ce qu'**il comprend :
- la mise en oeuvre d'un procédé de culture *in vitro* de cellules selon la revendication 6,
- le cas échéant, une ou plusieurs étapes de lavage des cellules en culture à l'aide d'une solution tampon appropriée, afin d'éliminer en tout ou partie le ou les inhibiteurs de LO utilisés,
- le cas échéant une étape de digestion enzymatique de la matière extracellulaire susceptible de s'être formée, à l'aide d'enzymes appropriées,
- le cas échéant une étape de récupération des cellules mises en culture.

8. Procédé de préparation *in vitro* d'une matrice cellulaire telle que définie dans la revendication 1, susceptible de pouvoir être utilisée en tant que tissus ou implants tissulaires, constitués de cellules différenciées au phénotype identique, **caractérisé en ce qu'**il comprend :
- la mise en oeuvre d'un procédé de culture *in vitro* de cellules selon la revendication 6, jusqu'à la formation d'une matrice cellulaire telle que définie ci-dessus, suffisante pour constituer une trame tissulaire,
- le cas échéant, une ou plusieurs étapes de lavage de la matrice cellulaire en culture à l'aide d'une solution tampon appropriée, afin d'éliminer en tout ou partie le ou les inhibiteurs de LO utilisés,
- le cas échéant une étape de récupération de la matrice cellulaire telle que définie ci-dessus.

9. Procédé de préparation *in vitro* de tissus selon la revendication 8, appliqué à la néosynthèse d'implants de cartilage lorsque ledit procédé est effectué à partir de chondrocytes, ou à la préparation de substitut cutanés lorsque ledit procédé est effectué à partir de fibroblastes et/ou de kératinocytes.

10. Procédé de criblage de molécules d'intérêt pharmacologique, notamment de médicaments, **caractérisé en ce qu'**il comprend :
- la mise en oeuvre d'un procédé de *culture in vitro* de cellules selon la revendication 6, le cas échéant jusqu'à la formation d'une matrice cellulaire suffisante pour constituer une trame tissulaire,
- le cas échéant, une ou plusieurs étapes de lavage des tissus en culture à l'aide d'une solution tampon appropriée, afin d'éliminer en tout ou partie le ou les inhibiteurs de LO utilisés,
- une étape de mise en présence de la molécule testée avec les cellules ou tissus obtenus lors des étapes précédentes,
- la détection de l'éventuel effet de ladite molécule sur les cellules ou les tissus susmentionnés.

11. Implants pour la thérapie cellulaire correspondant à des implants de suspensions de chondrocytes différenciés, dont le phénotype initial de type cartilage a été maintenu pendant la phase préliminaire de multiplication cellulaire *in vitro,* tels qu'obtenus par mise en oeuvre d'un procédé selon la revendication 7 **.**

12. Implants pour la thérapie tissulaire de type cartilage tels qu'obtenus par mise en oeuvre d'un procédé selon la revendication 8 effectué à partir de chondrocytes, comprenant une matrice extracellulaire exempte de collagènes normalement absents du cartilage sain (à savoir les collagènes I et III), et comprenant la totalité des collagènes spécifiques du cartilage (à savoir les collagènes II, IX, XI, et le cas échéant X).

13. Implants de cartilage selon la revendication 12, **caractérisés en ce qu'**ils contiennent des inhibiteurs de LO à l'état de traces.

14. Implants de peau reconstituée, ou substitut cutanés, tels qu'obtenus par mise en oeuvre d'un procédé selon la revendication 10 effectué à partir de fibroblastes et/ou de kératinocytes, la surface desdits implants étant diminuée de 40 % après 35 jours de culture par rapport à la surface de départ.

## Claims

1. Use of direct lysyl oxidase (LO) inhibitors, chosen among:
the primary amines reacting with the carbonyl group of the active site of the LOs, and more particularly those which produce, after binding with the carbonyl, a product stabilized by resonance, such as the following primary amines:
- ethylenediamine,
- aminonitriles, such as β-aminopropionitrile (β-APN), or 2-nitroethylamine,
- unsaturated or saturated haloamines, such as 2-bromo-ethylamine, 2-chloroethylamine, 2-trifluoroethylamine, 3-bromopropylamine, p-halobenzylamines, and unsaturated halogen compounds,
- selenohomocysteine lactone,
as inhibitors of the dedifferentiation of cells in culture *in vitro,* in order to keep constant the phenotype of said cells in the context of the implementation of a method of *in vitro* culture of the latter, and for the whole of the culture period, for the preparation of differentiated cells the phenotype of which is identical, or for the preparation of a cellular matrix constituted by differentiated cells preserving the same phenotype and cultured in the presence of said inhibitors, and of the extracellular medium secreted by said cells and binding the latter in said matrix.

2. Use of β-APN as LO inhibitor according to claim 1.

3. Use of LO inhibitors according to one of claims 1 or 2, for the implementation of methods of *in vitro* culture of all cells of human or animal origin, in which said cells are maintained in a phenotype with constant differentiation, and are chosen in particular from the chondrocytes of the cartilages, cornea cells, skin cells (such as the dermal fibroblasts, and epidermal keratinocytes), endothelial cells of the vessels, bone osteoblasts, hepatocytes, renal cells, muscle cells, stem or pluripotential cells.

4. Use of LO inhibitors according to one of claims 1 to 3, for the implementation of methods of *in vitro* culture of cells.

5. Use of LO inhibitors according to one of claims 1 to 3, for the implementation of methods of *in vitro* culture of cells in order to obtain tissues, such as skin or cartilage tissues.

6. Method of *in vitro* culture of cells as defined in claim 3, during which the phenotype of said cells is maintained at a stage identical to that in which said cells were found initially during their culture, said method comprising the culture of said cells in an appropriate medium containing one or more LO inhibitors defined in one of claims 1 to 2.

7. Method for preparing cells, or cell implants, **characterized in that** it comprises:
- the implementation of an *in vitro* cell culture method according to claim 6,
- if appropriate, one or more stages of washing the cells in culture using an appropriate buffer solution, in order to completely or partially eliminate the LO inhibitor or inhibitors used,
- if appropriate, a stage of enzymatic digestion of the extracellular material capable of being formed, using appropriate enzymes,
- if appropriate, a stage of recovery of the cells cultured.

8. Method for preparing *in vitro* a cell matrix as defined in claim 1, capable of being used as tissues or tissue implants, constituted by differentiated cells with identical phenotype, **characterized in that** it comprises:
- the implementation of an *in vitro* cell culture method according to claim 6, until formation of a cell matrix as defined above, which is sufficient to constitute a tissue stroma,
- if appropriate, one or more stages of washing of the cell matrix in culture using an appropriate buffer solution, in order to completely or partially eliminate the LO inhibitor or inhibitors used,
- if appropriate, a stage of recovery of the cell matrix as defined above.

9. Method for preparing *in vitro* tissues according to claim 8, used for the neosynthesis of cartilage implants when said method is carried out from chondrocytes, or
for the preparation of cutaneous substitutes when said method is carried out from fibroblasts and/or keratinocytes.

10. Method for screening molecules of pharmacological interest, in particular medicaments, **characterized in that** it comprises:
- the implementation of an *in vitro* cell culture method according to claim 6, if appropriate, until the formation of a cell matrix which is sufficient to constitute a tissue stroma,
- if appropriate, one or more stages of washing the tissues in culture using an appropriate buffer solution, in order to completely or partially eliminate the LO inhibitor or inhibitors used,
- a stage of placing the molecule tested in the presence of the cells or tissues obtained during the preceding stages,
- the detection of any effect of said molecule on the above-mentioned cells or tissues.

11. Implants for cellular therapy, corresponding to implants of suspensions of differentiated chondrocytes, the initial cartilage-type phenotype of which, was maintained during the preliminary phase of cell multiplication *in vitro,* as obtained by the implementation of a method according to claim 7.

12. Implants for tissular therapy of the cartilage type as obtained by implementation of a method according to claim 8 carried out from chondrocytes, comprising an extracellular matrix which is essentially free from collagens which are normally absent from healthy cartilage (namely collagens I and III), and comprising all of the collagens specific to cartilage (namely the collagens II, IX, XI, and if appropriate X).

13. Cartilage implants according to claim 12, **characterized in that** they contain LO inhibitors in trace amount.

14. Reconstituted skin implants, or cutaneous substitute, as obtained by implementation of a method according to claim 10 carried out from fibroblasts and/or keratinocytes, the area of said implants being diminished by 40% after 35 days of culture compared with the starting area.

## Patentansprüche

1. Verwendung von direkten Inhibitoren der Lysyloxidasen (LO), ausgewählt aus:
den primären Aminen, die mit der Carbonylgruppe der Wirkstelle der LO reagieren, und insbesondere jene,
die nach der Bindung mit dem Carbonyl ein resonanzstabilisiertes Produkt ergeben, wie etwa die folgenden primären Amine:
- Ethylendiamin,
- Aminonitrile, wie etwa β-Aminopropionitril (β-APN) oder 2-Nitroethylamin,
- gesättigte oder ungesättigte Haloamine, wie etwa 2-Bromethylamin, 2-Chlorethylamin, 2-Trifluorethylamin, 3-Brompropylamin, p-Halobenzylaminen und ungesättigte halogenierte Verbindungen,
- Selenohomocysteinlacton,
als Inhibitoren der Entdifferenzierung der Zellen in In-vitro-Kultur zum konstanten Erhalt eines Phänotyp der Zellen im Rahmen der Durchführung eines Verfahrens zur *In-vitro*-Zellkultur, und zwar über die gesamte Dauer der Kultur hinweg, zur Herstellung von differenzierten Zellen, deren Phänotyp identisch ist, oder zur Herstellung einer Zellmatrix, die aus differenzierten Zellen, deren Phänotyp konserviert ist und die in Gegenwart der Inhibitoren kultiviert wurden, und aus extrazellulärem Medium besteht, das von den Zellen sekretiert wird und diese Zellen in der Matrix bindet.

2. Verwendung von β-APN als LO-Inhibitor.

3. Verwendung von LO-Inhibitoren nach einem der Ansprüche 1 bis 2 zum Durchführen von Verfahren zur *In-vitro-*Kultur aller Zellen menschlichen oder tierischen Ursprungs, bei denen die Zellen mit einem konstanten Differenzierungsphänotyp erhalten werden, und die insbesondere ausgewählt sind aus Chondrocyten der Knorpel, Hornhautzellen, Hautzellen (wie etwa Fibroblasten der Dermis und Keratinocyten der Epidermis), Endothelzellen der Gefäße, Osteoblasten der Knochen, Hepatocyten, Nierenzellen, Muskelzellen, Stammzellen oder pluripotenten Zellen.

4. Verwendung von LO-Inhibitoren nach einem der Ansprüche 1 bis 3 zum Durchführen von In-vitro-Kulturverfahren.

5. Verwendung von LO-Inhibitoren nach einem der Ansprüche 1 bis 3 zum Durchführen von In-vitro-Zellkulturen zum Erhalt von Geweben, wie etwa Haut oder Knorpel.

6. Verfahren zur *In-vitro*-Zellkultur, wie in Anspruch 3 definiert, in dessen Verlauf der Phänotyp der Zellen in einem Stadium erhalten wird, das mit dem identisch ist, in dem sich die Zellen anfänglich bei ihrer Kultivierung befanden, wobei das Verfahren die Kultivierung der Zellen in einem geeigneten Medium umfasst, das einen oder mehrere LO-Inhibitoren enthält, die in einem der Ansprüche 1 bis 2 definiert wurden.

7. Verfahren zur Herstellung von Zellen oder Zellimplantaten, **dadurch gekennzeichnet, dass** es Folgendes umfasst:
- Durchführen eines Verfahren zur *In-vitro*-Zellkultur nach Anspruch 6,
- gegebenenfalls einen oder mehrere Schritte zum Waschen der Kulturzellen mit Hilfe einer geeigneten Pufferlösung, um den oder die verwendeten LO-Inhibitoren ganz oder teilweise zu entfernen,
- gegebenenfalls einen Schritt zum enzymatischen Abbau der extrazellulären Substanz, die sich gebildet haben kann, mit Hilfe geeigneter Enzyme,
- gegebenenfalls einen Schritt zur Gewinnung der kultivierten Zellen.

8. Verfahren zum Herstellen einer Zellmatrix *in vitro,* wie in Anspruch 1 definiert, die als Gewebe oder Gewebeimplantate verwendet werden kann, die aus differenzierten Zellen mit identischem Phänotyp bestehen, **dadurch gekennzeichnet, dass** es Folgendes umfasst:
- Durchführen eines Verfahrens zur *In-vitro*-Zellkultur nach Anspruch 6 bis zur Bildung einer Zellmatrix, wie oben definiert, die ausreicht, um ein Grundgewebe zu bilden,
- gegebenenfalls einen oder mehrere Schritte zum Waschen der Zellmatrix mit Hilfe einer geeigneten Pufferlösung, um den oder die verwendeten LO-Inhibitoren ganz oder teilweise zu entfernen,
- gegebenenfalls einen Schritt zum Gewinnen der Zellmatrix, wie oben definiert.

9. Verfahren zur Herstellung von Geweben *in vitro* nach Anspruch 8, das auf die Neosynthese von Knorpelimplantaten angewendet wird, wenn das Verfahren ausgehend von Chondrocyten ausgeführt wird, oder auf die Herstellung von Hautsubstituten, wenn das Verfahren ausgehend von Fibroblasten und/oder Keratinocyten ausgeführt wird.

10. Verfahren zum Screenen von Molekülen von pharmakologischem Interesse, insbesondere von Medikamenten, **dadurch gekennzeichnet, dass** es Folgendes umfasst:
- Durchführen eines Verfahrens zur *In-vitro-*Zellkultur nach Anspruch 6, gegebenenfalls bis zur Bildung einer Zellmatrix, die ausreicht, um ein Grundgewebe zu bilden,
- gegebenenfalls einen oder mehrere Schritte zum Waschen der Kulturgewebe mit Hilfe einer geeigneten Pufferlösung, um den oder die verwendeten LO-Inhibitoren ganz oder teilweise zu entfernen,
- einen Schritt zum Mischen des getesteten Moleküls mit den Zellen oder Geweben, die in den vorhergehenden Schritten erhalten wurden,
- Nachweisen der möglichen Wirkung des Moleküls auf die oben genannten Zellen oder Gewebe.

11. Implantate für die Zelltherapie, die Implantaten aus Suspensionen differenzierter Chondrocyten entsprechen, wobei der Knorpeltyp während einer vorausgehenden Phase der In-vitro-Zellvermehrung erhalten wurde, wie durch die Durchführung eines Verfahrens nach Anspruch 7 erhalten.

12. Implantate für die Gewebetherapie vom Typ Knorpelgewebe, wie durch die Durchführung eines Verfahrens nach Anspruch 8 erhalten, die ausgehend von Chondrocyten ausgeführt wurden, die eine extrazelluläre Matrix umfassen, die frei von Kollagenen ist, die gesundem Knorpel normalerweise fehlen (nämlich die Kollagene I und III), und die Gesamtheit der spezifischen Kollagene des Knorpels (nämlich die Kollagene II, IX, XI und gegebenenfalls X) umfassen.

13. Knorpelimplantate nach Anspruch 12, **dadurch gekennzeichnet, dass** sie LO-Inhibitoren in Form von Spuren enthalten.

14. Implantate aus rekonstituierter Haut oder Hautsubstitute, wie durch die Durchführung eines Verfahrens nach Anspruch 10 erhalten, die ausgehend von Fibroblasten und/oder Keratinocyten realisiert wurden, wobei die Oberfläche der Implantate nach 35 Tagen Kultur bezogen auf die Ausgangsoberfläche um 40 % verringert ist.
